(19) Europäisches Patentamt
European Patent Office
Office européen des brevets

(11) **EP 2 711 758 A1**

(12) **EUROPÄISCHE PATENTANMELDUNG**

(43) Veröffentlichungstag:
26.03.2014  Patentblatt 2014/13

(51) Int Cl.:
**G02B 21/00** (2006.01)  **A61B 5/00** (2006.01)
**G02B 21/34** (2006.01)

(21) Anmeldenummer: **12185535.7**

(22) Anmeldetag: **21.09.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Mavig GmbH**
**81829 München (DE)**

(72) Erfinder: **Irmer, Jochen**
**82335 Bachhauser Wies (DE)**

(74) Vertreter: **Stolmár & Partner**
**Blumenstraße 17**
**80331 München (DE)**

(54) **Markierungsaufsatz für konfokale Mikroskope**

(57) Die Erfindung betrifft einen Markierungsaufsatz (MA) für ein konfokales Mikroskop (10), ein konfokales Mikroskop (10) und ein Verfahren zum Betreiben des Mikroskops (10). Dabei kann das Mikroskop (10) in einem Aufnahmemodus und in einem Markierungsmodus betrieben werden, wobei der Wechsel zwischen den beiden Modi durch eine Bewegung des Objektivhalters (H) ausgelöst wird. Der Markierungsaufsatz umfasst eine Platine (3) mit einem Beschleunigungssensor (4) und einer Aktivierungseinheit (5) und einem Laserpointer (2), der zur Markierung einer Zielposition (Z) dient. Dabei ist der Lichtsignalgeber (2) so auf dem Markierungsaufsatz (MA) in Bezug auf das Objektiv des Mikroskops (10) angeordnet, dass der Lichtsignalgeber (2) im Markierungsmodus exakt den Konfokalpunkt des Mikroskopobjektivs trifft.

**Fig. 2**

**Beschreibung**

GEBIET DER ERFINDUNG

Die vorliegende Erfindung liegt auf den Gebieten der Physik und Elektronik sowie der bildgebenden Medizintechnik. Die Erfindung betrifft insbesondere die konfokale Mikroskopie und eine Referenzierung der dabei erfassten Bilddarstellungen (das mikroskopische Bild wird üblicherweise auf einem Monitor dargestellt) mit Positionen auf der Oberfläche des zu untersuchenden Objektes, wie z.B. der Haut eines Patienten bei der Erkennung von Hautkrebs oder anderen Hautkrankheiten.

Die konfokale Mikroskoptechnik bietet einen geeigneten Ansatz, um Hautkrebs (auch im Frühstadium) zu erkennen. Bei einem konfokalen Lasermikroskop dient der Laser als Lichtquelle mit mehreren Linien zur Anregung der Fluoreszenzfarbstoffe. Ein Scanner enthält eine konfokale Pinhole-Anordnung (Lochblende) und einen Strahlteiler, der das Anregungslicht reflektiert und das zu detektierende Fluoreszenzlicht durchlässt. Das Anregungslicht wird durch einen beweglichen Spiegel rasterförmig über das unter dem Mikroskop befindliche zu untersuchende Objekt (z.B. Haut) bewegt. Ein Detektor weist das vom Objekt reflektierte oder emittierte Fluoreszenzlicht nach Farben selektiert nach. Da man lediglich Licht von einem Punkt des Objekts erhält, ist es notwendig die Probe abzurastern und das Bild am Computer zu rekonstruieren. Das gesamte System wird von einem Rechner gesteuert. Das konfokale Prinzip dieser Anordnung basiert im Wesentlichen darauf, dass Informationen, die nicht aus der Bildebene des Mikroskops stammen, vor dem Detektor ausgeblendet werden. In der Regel ist der Lichtdetektor als Photomultiplier ausgebildet. Optische Information, die nicht aus der Fokalebene kommt, wird somit nicht erfasst. Hierdurch erhält das konfokale Mikroskop seine einzigartige Eigenschaft "optische Schnitte" durch das zu untersuchende Objekt oder durch die Probe zu legen. Unter den hauptsächlich im Einsatz befindlichen konfokalen Laser-Scanning-Mikroskopen sind Punktscanner, bei denen ein fokussierter Laserstrahl das Präparat abrastert oder Linienscannern bekannt (bei denen eine ganze Bildzeile auf einmal und somit insgesamt schneller erstellt wird).

Der Aufbau und die Funktionsweise eines konfokalen Mikroskops ist im Stand der Technik beispielsweise bekannt aus der internationalen Anmeldung PCT/US2006/018777.

STAND DER TECHNIK

In vielen Anwendungsfällen, wie z.B. einem operativen Eingriff unter Verwendung eines Mikroskops aber auch bei einer Untersuchung von natürlichen oder technischen Proben erweist es sich grundsätzlich als wesentlich, den auf dem Monitor abgebildeten Bildausschnitt dem untersuchten Hautoberflächensegment zuzuordnen und umgekehrt. Dies ist bei einem operativen Eingriff beispielsweise in der Praxis sehr wichtig, da der Arzt bei einer auszuführenden Untersuchung oder bei einem operativen Eingriff sehr genau zwischen benignem und malignem Gewebe differenzieren muss. Dazu dient zum Einen der rekonstruierte Bildausschnitt auf dem Monitor. Zum Anderen sind jedoch auch mit dem bloßen Augen erkennbare Hinweise (möglicherweise auch in der Umgebung des untersuchten Hautsegmentes) auf der Haut des Patienten hilfreich. Dazu muss die das Mikroskop anwendende Person jedoch wissen, welche Stelle abgetastet worden ist, um diese direkt zu bewerten und zu analysieren. Da in der Regel nur einzelne Punkte gescannt werden können, sind mehrere Aufnahmen notwendig. Für jede dieser Aufnahmen soll es möglich sein, eine Zuordnung auf der Haut zu finden.

Dazu ist es aus dem Stand der Technik beispielsweise bekannt, Markierungsdaten auf der Haut eines Patienten aufzubringen, nachdem die Hautstelle abgetastet worden ist. So zeigt die WO 00/15105 einen Ansatz, mit dem Markierungen durch zusätzliche Mittel (Druckkopf) auf die Haut des Patienten aufgebracht werden können, nachdem die Haut mit einem mit einem Schrittmotor betriebenen Mikroskop belichtet worden ist. Die Markierungsmittel sind dabei als separates Bauteil vorgesehen und insbesondere separat zum Objektivhalter. Um die Positionsberechnung für den Druckkopf zur anschließenden Markierung auszuführen, wird die Bewegung des Mikroskopkopfs durch die Bewegung des Schrittmotors erfasst und in eine absolute Position umgerechnet. Diese berechneten Werte können dann an den Drucker oder einer anderen Markierungseinheit weitergegeben werden. Dieser Aufbau erhöht zum Einen nachteiligerweise die Kosten, erfordert eine zusätzliche Positionsberechnung und erschwert die Handhabung, da dies für einen mobilen Einsatz nicht anwendbar ist.

Als nachteilig erweist es sich des Weiteren, dass bei dem vorstehenden Verfahren eine Positionsberechnung notwendig war, die häufig zu Fehlern führte. Des Weiteren kann das vorstehende Verfahren nicht für mobile Handgeräte und nicht ohne Ringaufsatz verwendet werden, was den Anwendungsbereich für mögliche Untersuchungen einschränkt.

AUFGABE

Die vorliegende Erfindung hat sich deshalb zur Aufgabe gestellt, ein bekanntes konfokales Mikroskop dahingehend zu verbessern, dass eine Zuordnung zwischen vom Mikroskop gescannten Bildpunkten, die auf einem Monitor dargestellt werden einerseits und dem sichtbaren, untersuchten Bereich auf der Haut möglich wird und umgekehrt, ohne zusätzliche Berechnungen ausführen zu müssen und ohne das Mikroskop umrüsten oder von dem untersuchten Objekt wegbewegen zu müssen. Des Weiteren soll die Untersuchung mit einem konfokalen Mikroskop verbessert und flexibilisiert werden. Ebenso soll es möglich sein, den Objektivkopf anhand einer zuvor aufgebrachten Markierung oder Kennzeichnung auf der Haut (oder auf dem Objekt) zu positionieren. Umgekehrt soll es möglich sein, bei einem zuvor positionierten Objektivkopf des Mikroskops und einer dort ausgeführten Datenerfassung mit einer Darstellung der erfassten Bilddaten auf einem Monitor, anschließend die Position, die der Datenerfassung zugrunde lag eineindeutig auf der Haut zu kennzeichnen bzw. zu markieren. Dabei soll das Mikroskop weitestgehend stationär verbleiben und unverändert verwendbar sein, ohne dass es notwendig ist, Umrüstmaßnahmen auszuführen, indem beispielsweise separate Module und Einheiten installiert werden müssen.

BESCHREIBUNG DER ERFINDUNG

Diese Aufgabe wird mit einem Verfahren, einem Markierungsaufsatz und mit einem Mikroskop gemäß den beiliegenden Patentansprüchen gelöst.

Im Folgenden wird die Erfindung anhand des Aufsatzes beschrieben. Hierbei erwähnte Ausführungsformen, alternative Lösungen, weitere Merkmale und Vorteile sind ebenso auch auf die anderen Lösungen der vorstehend genannten Aufgabe zu übertragen (also auf das Verfahren bzw. auf das Gerät an sich) und umgekehrt. Demnach können auch die Merkmale, die im Zusammenhang mit dem Aufsatz oder dem Mikroskop beansprucht und/oder beschrieben sind, auch auf das Verfahren angewendet werden und umgekehrt. Dabei sind die jeweiligen funktionalen Merkmale des Verfahrens durch entsprechende Module, darunter auch Mikroprozessormodule oder Hardwaremodule, implementiert, die dazu ausgebildet sind, die jeweilige Funktionalität zu übernehmen.

Gemäß einem Aspekt bezieht sich die Erfindung auf Markierungsaufsatz für ein konfokales Mikroskop, der zum Eingriff mit einem Objektivhalter des konfokalen Mikroskops bestimmt ist, mit:

- Einer mechanischen Halterung, die zum Eingriff mit dem Objektivhalter derart bestimmt ist, dass der Markierungsaufsatz in einer vorbestimmten, ortsfesten Position mit dem Objektivhalter verrastet
- Zumindest einem Lichtsignaigeber, der jeweils in einem vordefinierten Winkel an dem Markierungssaufsatz angeordnet ist,
- Einer Platine mit einem elektronischen Schaltkreis mit:

　　o Einem drei-achsigen Lagesensor, der zur Lageerfassung des Markierungsaufsatzes in allen drei Raumachsen und daraus ableitbaren Geschwindigkeiten des Objektivhalters in allen drei Raumachsen bestimmt ist
　　o Aktivierungseinheit, die dazu bestimmt ist, ein Aktivierungssignal an den Lichtsignalgeber zur Aktivierung des Lichtsignalgebers zu senden, falls das erfasste Geschwindigkeitssignal einen vorkonfigurierbaren Schwellenwert übersteigt

Gemäß einem weiteren Aspekt bezieht sich die Erfindung auf ein konfokales Mikroskop mit einem Markierungsaufsatz.

Ein weiterer Aspekt der Erfindung betrifft Verfahren zum Betreiben eines konfokalen Mikroskops mit einem Objektivhalter in:

- einem Aufnahmemodus, in dem Beleuchtungsinformationen aus der Objektoberfläche an zumindest einer Zielposition abgetastet werden und zu einer auf einem Monitor darstellbaren Bilddarstellung rekonstruiert werden und

- einem Markierungsmodus, in dem eine Bilderfassung des konfokalen Mikroskops deaktiviert ist und in dem ein Lichtsignalgeber aktiviert ist, der die abgetastete Zielposition auf der untersuchten Objektoberfläche belichtet, wobei ein Wechsel zwischen Aufnahmemodus und Markierungsmodus des konfokalen Mikroskops durch eine schnelle Kipp- oder Schwenkbewegung des Objektivhalters erfolgt.

Im Folgenden werden die in dieser Anmeldung verwendeten Begrifflichkeiten der Erfindung näher erläutert.

Der Begriff "Objektivhalter" umfasst das Objektiv kann auch im Sinne eines Oberbegriffs zum Begriff "Objektiv" verstanden werden. Üblicherweise ist hierunter ein Arm zu verstehen, der das Objektiv trägt. Der Objektivhalter ist vorzugsweise als ein mobiles bewegliches Handgerät ausgebildet oder in ein solches integriert. Der Objektivhalter umfasst das Objektiv mit dem Objektivfenster, das mit einer Abdeckklappe geschützt sein kann, eine Haltevorrichtung zur Bedienung des Gerätes sowie Schnittstellen zum Datenaustausch und zur Stromversorgung. Der Objektivhalter ist vorzugsweise eine Trägerkonstruktion für das gesamte Mikroskop.

Der Markierungsaufsatz ist als vorwiegend flächiges Modul ausgebildet in einer annähernd rechteckförmigen Form mit geringer Höhenausdehnung. Die Höhe beträgt zwischen 3mm und 1 cm. Der Markierungsaufsatz ist vorzugsweise als separates Kunststoffmodul ausgebildet, das auf den Objektivhalter des Mikroskops aufgesetzt werden kann, um mit ihm in einer vordefinierten Position ortsfest zu verrasten. Der Markierungsaufsatz kann durch eine integrierte Batterie, durch Anschluss an die Spannungsversorgung des Mikroskops oder durch eine USB-Schnittstelle mit Spannung versorgt werden. Der Markierungsaufsatz kann als zum Objektivhalter separates Bauteil vorgesehen sein, dass bedarfsweise mit dem Mikroskop kombiniert (fest, aber lösbar) verbunden werden kann. Alternativ kann der Aufsatz auch in das Mikroskop, insbesondere im Schaftbereich des Objekthalters integriert werden. Die Auswerteelektronik kann dann direkt mit der Bilderfassungselektronik kombiniert werden.

"Ortsfest" meint in diesem Kontext eine Fixierung des Markierungsaufsatzes an dem Mikroskop, insbesondere an dem Objektivhalter, die lösbar ist (Schraubverbindung und/oder Steckverbindung). Des Weiteren ist die Verbindung in radialer Hinsicht, in axialer Hinsicht (bezogen auf eine Längsachse des Objektivhalters) und in Hinblick auf eine mögliche Rotation des Aufsatzes um die Längsachse des Objektivhalters fixiert. Vorstehende Bewegungsoptionen sind somit unterbunden.

Der vordefinierte Winkel, in dem der Stutzen (rohrförmig, umfassend die Laserquelle zur Markierung) an dem Markierungselement ausgebildet ist (vorzugsweise einstückig) beträgt in etwa 65°. Je nach Länge des Objektivhalters und abhängig von den weiteren geometrischen Verhältnissen kann der Winkel jedoch auch variieren. Wesentlich ist, dass der Winkel so gewählt wird, dass der Lichtsignalgeber im Markierungsmodus den Markierungsstrahl in einer Markierungsposition auf der Hautoberfläche trifft, die exakt mit der Konfokalposition des Konfokalstrahls im Konfokalmodus übereinstimmt, so dass durch eine geometrische oder mechanische Anordnung (und somit Kopplung) eine örtliche Referenzierung zwischen Markierungsstrahl und Konfokalstrahl bereitgestellt werden kann (keine zeitliche, da ein Markierungsmodul nicht zeitgleich mit einem Konfokal-oder Aufnahmemodus ausgeführt werden kann). Die Platine ist eine Leiterplatte mit elektronischen Bauteilen, umfassend den Lage - oder Beschleunigungssensor, der auch als Gyratorbaustein ausgebildet sein kann und die Aktivierungseinheit. Darüber hinaus kann die Platine noch weitere Bauteile, wie z.B. eine Prozessor und Speicherbausteine umfassen.

Das Verfahren kennt zwei Betriebsmodi: einen Markierungsmodus und einen Aufnahme- oder auch Konfokalmodus genannt. Zwischen den beiden Modi kann gewechsel werden, in dem das Mikroskop auf eine vordefinierte Weise (umfassend eine allgemeine Lageveränderung und eine schnelle Kipp- oder Schwenkbewegung) bewegt wird. Eine zusätzliche Berechnung von Positionen oder ein Gerätewechsel oder ein Umrüsten des Mikroskops ist dazu nicht erforderlich. Gemäß einer bevorzugten Ausführungsform der Erfindung ist es vorgesehen, dass beim Übergang vom Konfokalmodus in den Markierungsmodus der Laser erst angeschaltet wird wenn der Markierungsaufsatz den korrekten Winkel erreicht hat und tatsächlich den Konfokalpunkt des Mikroskops trifft.

Das Verfahren kann in einem Suchbetrieb verwendet werden. Dazu wird zunächst ein Zielpunkt auf der Haut mit dem Konfokallaser abgetastet und auf dem Monitor dargestellt. Nun besteht die Aufgabe darin, den auf dem Monitor angezeigten Punkt auch auf der realen Hautoberfläche "wiederzufinden". Dazu wird der Objekthalter einfach an der Zielposition gekippt oder abgeklappt, so dass der Objektivhalter in die Markierungsposition überführt wird. Diese ist vordefiniert und vorzugsweise dadurch definiert, dass die Längsachse des Stutzens senkrecht auf der Hautoberfläche auf die Zielposition zeigt. Nun kann der Lichtsignalgeber zur Markierung aktiviert werden.

Das Verfahren kann auch in einem zweiten Positionierbetrieb verwendet werden. Dazu wird zunächst ein Zielpunkt mit dem Lichtsignalgeber belichtet, so dass die Zielposition auf der Hautoberfläche markiert werden kann (zum Beispiel von Hand - ist aber optional). Nun besteht die Aufgabe darin, an genau diesem belichteten oder markierten Zielpunkt eine konfokale Aufnahme zu erfassen. Dazu wird das Gerät aus dem Markierungsmodus durch eine Kippbewegung in den Aufnahmemodus überführt. Dazu wird der Objektivhalter aus der Markierungsposition in eine Konfokalposition bewegt. Sobald diese eingenommen ist, kann optional der konfokale Laserstrahl aktiviert werden oder aktiviert bleiben. Die Konfokalposition ist vordefiniert. Sie kennzeichnet sich vorzugsweise dadurch, dass die Längsachse des Objektivhalters lotrecht auf der Hautoberfläch steht.

Der Aufnahmemodus wird aktiviert, indem der Anwender das Mikroskop neigt ohne, dass es auf der Hautoberfläche ist. Der Anwender sucht nun den jeweiligen point of interest ('POI'), der vom Laserpointer angezeigt wird und setzt das Objektiv auf die Hautoberfläche. Optional kann eine zweite Anzeigeeinheit (z.B. in Form eines optischen Signalgebers, wie einer LED) vorgesehen sein, die den korrekten Winkel anzeigt bzw. bei Positionierung im korrekten (vorbestimmten Winkel) ein grünes Signal ausgibt. Durch Kippen des Objektivhalters auf die Hautoberfläche kann daraufhin in den Konfokalmodus gewechselt werden.

Ein wesentliches Merkmal der Erfindung ist in der optischen Referenzierung zu erkennen. Mit der Erfindung wird es möglich, die auf dem Monitor rekonstruierten Mikroskop-Bilddaten mit der realen Haut- oder Objektoberfläche zu registrieren. Dabei entspricht eine Referenzposition in dem Mikroskop Bild, die z.B. durch ein Fadenkreuz gekennzeichnet werden kann, einer mit dem Lichtsignalgeber zum Zwecke der Markierung belichteten Zielposition auf der Haut. Der Arzt kann somit auf einen Blick erkennen, wo er sich gerade befindet.

Die Lageveränderung des Objektivhalters zum Wechseln des Betriebsmodus in den Aufnahmemodus ist vorzugsweise unabhängig von einer Geschwindigkeit der Objektivhalterbewegung (ob schnell oder langsam). Sobald erfasst ist, dass der Objektivhalter in eine Aufnahmeposition bewegt ist (vorkonfiguriert: 90° zur Hautoberfläche) erfolgt ein automatisches Aktivieren des konfokalen Bildinhalts.

Der Lichtsignalgeber kann als Laserpointer ausgebildet werden.

Das Mikroskop ist vorzugsweise als Handgerät ausgebildet und in allen Raumachsen von Hand oder über eine Apparatur bewegbar.

Die Berechnungsformeln für den Raumwinkel, in dem der Lichtsignalgeber an dem Markierungsaufsatz befestigt ist, lauten in der bevorzugten Ausführungsform der Erfindung:

Für den Cosinus-Anteil:

$$\cos\alpha = \frac{a_x}{\sqrt{a_x^2 + a_y^2 + a_z^2}}$$

$$\cos\beta = \frac{a_y}{\sqrt{a_x^2 + a_y^2 + a_z^2}}$$

$$\cos\gamma = \frac{a_z}{\sqrt{a_x^2 + a_y^2 + a_z^2}}$$

Für den Sinus-Anteil:

$$\sin\theta = \frac{\sqrt{a_y^2 + a_z^2}}{\sqrt{a_x^2 + a_y^2 + a_z^2}}$$

$$\sin\phi = \frac{\sqrt{a_x^2 + a_z^2}}{\sqrt{a_x^2 + a_y^2 + a_z^2}}$$

Die allgemeine Rotation A ist beschrieben als ein Produkt aus drei Verschiebungsmatrixen: A=BCD mit:

$$\mathbf{B} = \begin{bmatrix} \cos\psi & \sin\psi & 0 \\ -\sin\psi & \cos\psi & 0 \\ 0 & 0 & 1 \end{bmatrix}$$

$$\mathbf{C} = \begin{bmatrix} 1 & 0 & 0 \\ 0 & \cos\theta & \sin\theta \\ 0 & -\sin\theta & \cos\theta \end{bmatrix}$$

$$\mathbf{D} = \begin{bmatrix} \cos\varphi & \sin\varphi & 0 \\ -\sin\varphi & \cos\varphi & 0 \\ 0 & 0 & 1 \end{bmatrix}$$

Die vorstehend beschriebenen Ausführungsformen des Verfahrens beziehen sich auf eine Hardwareimplementierung mit einer Platine und integrierten Schaltkreisen. Alternativ ist es auch möglich, die Routinen als Softwareroutinen zu implementieren mit entsprechenden Schnittstellen zu Messsensoren (Beschleunigungssensor, Aktivierungseinheit und Lichtsignalgeber etc.).

Es liegt im Rahmen der Erfindung, dass nicht alle Schritte des Verfahrens zwangsläufig auf ein und derselben Instanz ausgeführt werden müssen, sondern sie können auch auf unterschiedlichen Instanzen oder Bauteilen ausgeführt werden. Auch kann die Abfolge der Verfahrensschritte gegebenenfalls variiert werden.

Darüber hinaus ist es möglich, dass die Markierungseinheit nicht als separate und lösbare Einheit bereit gestellt wird, sondern direkt in das Mikroskop integriert wird.

KURZE BESCHREIBUNG DER FIGUREN

In der folgenden detaillierten Figurenbeschreibung werden nicht einschränkend zu verstehende Ausführungsbeispiele mit deren Merkmalen und weiteren Vorteilen anhand der Zeichnung besprochen.

Es zeigen:

Figur 1      eine schematische, übersichtsartige Darstellung eines konfokalen Mikroskops mit einem Markierungsaufsatz und einem Monitor zur Darstellung der erfassten Bilddaten gemäß einer bevorzugten Ausführungsform der Erfindung,

Figur 2      eine schematische Darstellung eines Markierungsaufsatzes gemäß einer bevorzugten Ausführungsform der Erfindung,

Figur 3      eine schematische Darstellung des Markierungsaufsatzes in einem Markierungsmodus,

Figur 4 eine schematische Darstellung des Markierungsaufsatzes in einem Konfokal- bzw. Aufnahmemodus

Figur 5 ein Ablaufdiagramm zur Ausführung eines Verfahrens für einen Markierungsbetrieb,

Figur 6 ein Ablaufdiagramm gemäß einer Ausführungsform der Erfindung für einen Aufnahmebetrieb und

Figur 7 eine schematische, übersichtsartige Darstellung des Markierungsaufsatzes mit weiteren Bauteilen, dabei zeigt Figur 7a eine Draufsicht auf den Markierungsaufsatz und Figur 7b eine Seitenansicht gemäß der Linie A-A in Fig. 7a.

Figur 8 eine schematische Zeichnung eines Ausschnittes des Markierungsaufsatzes mit einer Winkelangabe

## DETAILLIERTE BESCHREIBUNG DER FIGUREN UND EINZELNE AUSFÜHRUNGSBEISPIELE

Die Erfindung betrifft einen Markierungsaufsatz MA für ein konfokales Mikroskop 10, ein konfokales Mikroskop 10, und ein Verfahren zum Betreiben des konfokalen Mikroskopes 10.

Die hauptsächliche Anwendungsform betrifft konfokale Laser-Scanning-Mikroskope. Es ist jedoch ebenso möglich, andere bildgebende Geräte mit dem Markierungsaufsatz MA auszubilden, um eine Markierungsfunktion bereitstellen zu können. Üblicherweise ist das Konfokalmikroskop als mobiles Handgerät ausgebildet und kann - beispielsweise von einem behandelnden Arzt - in der Hand gehalten werden. Darüber hinaus sind auch chirurgische Anwendungsfälle denkbar, bei denen das Mikroskop im Rahmen des operativen Eingriffes eingesetzt wird. Der Begriff "konfokales Mikroskop", wie er im Rahmen dieser Anmeldung verwendet wird, kann somit auch noch weiter verstanden werden, so dass er noch andere mobile (d.h. bewegliche) bildgebende (insbesondere medizinische) Geräte umfasst.

Ein konfokales Laser-Rastermikroskop 10 fokussiert den Anregungslaserstrahl mit einem beweglichen Spiegel- und Pinhole-System über einer Zielposition Z auf einer Oberfläche eines zu untersuchenden Objektes. Je nach Anwendung ist es möglich, eine zweidimensionale Abbildung der aufgezeichneten Schnitte abzubilden und auf einem Monitor M darzustellen oder eine dreidimensionale Rekonstruktion des untersuchten Objektes darzustellen, indem mehrere Schnitte in unterschiedlichen Fokusebenen aufgezeichnet werden.

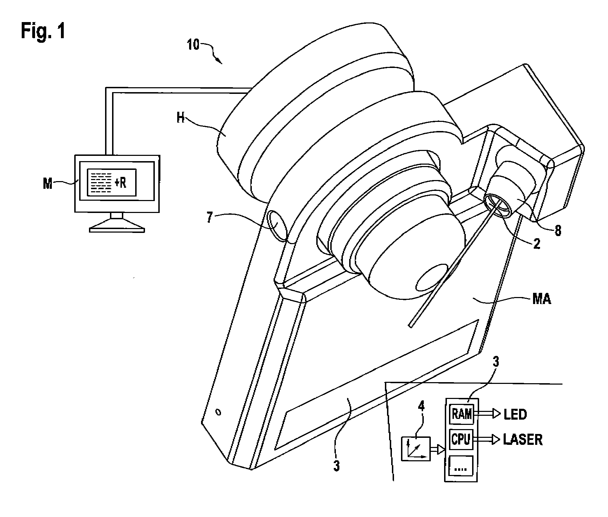

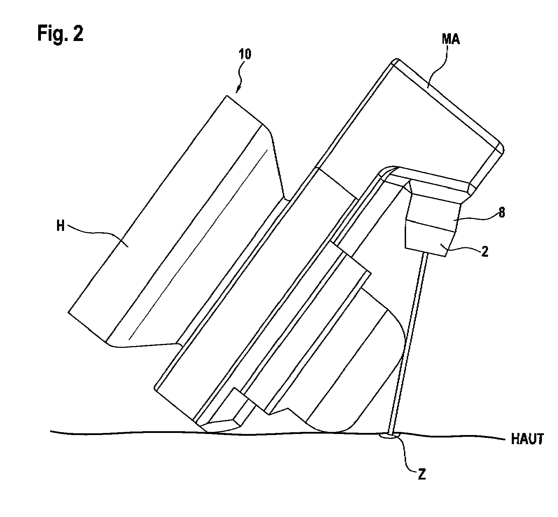

Wie in **Fig. 1** schematisch dargestellt, ist der Markierungsaufsatz MA an einem Objektivhalter H des konfokalen Mikroskops 10 ortsfest befestigt. Der Objektivhalter H umfasst das Objektiv, den Markierungsaufsatz MA und möglicherweise weitere Bauteile und Einheiten, sowie eine Spannungsversorgung und mögliche Schnittstellen zum Datenaustausch. Das konfokale Mikroskop wird auf der Oberfläche des zu untersuchenden Objektes aufgesetzt und dort langsam bewegt. Dabei steht eine Längsachse des Objektivhalters senkrecht auf einer Oberfläche des zu untersuchenden Objektes. Sequentiell werden Punkte der Oberfläche, zum Beispiel der Haut eines Patienten, abgetastet und über eine Schnittstelle an den Monitor M übertragen. Auf dem Monitor M wird die rekonstruierte Bilddarstellung visualisiert. Eine Kontrolleinheit 30, die in Fig. 1 nur schematisch dargestellt ist, dient dazu, Lasersteuersignale und/oder LED Steuersignale an den Markierungsaufsatz MA und/oder an den Objektivhalter H (und somit an das Mikroskop 10) zu senden. Die Kontrolleinheit 30 kann in dem Markierungsaufsatz oder als separates Bauteil oder integriert in das Mikroskop bereitgestellt werden. Die Kontrolleinheit 30 ist in der bevorzugten Ausführungsform der Erfindung als Platine 3 ausgebildet, die weiter unten in Zusammenhang mit Fig. 2 näher beschrieben ist.

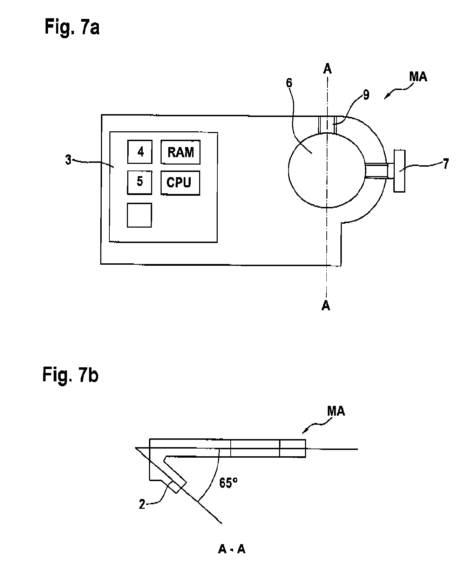

Unter Bezugnahme auf **Fig. 2** wird im Folgenden der grundsätzliche Aufbau des Markierungsaufsatzes MA gemäß einer bevorzugten Ausführungsform näher beschrieben. Der Markierungsaufsatz weist in der Draufsicht eine im Wesentlichen rechteckförmige Gestalt auf und dient zum Eingriff mit dem Objektivhalter H des konfokalen Mikroskops 10. Der Markierungsaufsatz wird vorzugsweise in einer vordefinierten Position auf dem Objektivhalter H befestigt oder verrastet. Dazu umfasst der Markierungsaufsatz eine mechanische Halterung 1, die vorzugsweise als kreisförmige Aufnahmeöffnung 6 ausgebildet ist, die einen lichten Durchmesser aufweist, der in etwa dem Außendurchmesser des Objektivhalters H an der Verrastungsposition entspricht. Dies hat den Vorteil, dass der Markierungsaufsatz MA leicht und schnell (eigentlich nur durch zwei Handgriffe) an dem konfokalen Mikroskop 10 ortsfest befestigt werden kann. Zunächst wird der Markierungsaufsatz MA auf den Objektivhalter H bis zum Anschlag aufgeschoben. Dann wird ein Verriegelungsmechanismus 7 bedient, um den Markierungsaufsatz auf dem Objektivhalter ortsfest zu positionieren. "Ortsfest" bedeutet in diesem Zusammenhang eine fixierte Position des Markierungsaufsatzes MA auf dem Objektivhalter H im Hinblick auf eine axiale Bewegung (bezogen auf die Längsachse des Objektivhalters H), auf eine radiale Bewegung des Markierungsaufsatzes MA und auf eine umfangsbezogene Drehbewegung des Markierungsaufsatzes MA bezogen auf den Schaft des Objektivhalters H. Zur zusätzlichen Fixierung bzw. Feinjustierung kann der Markierungsaufsatz MA zusätzlich noch eine Verstellschraube 9 umfassen, die beispielsweise als kleine Inbusschraube ausgebildet sein kann, um die Position des Markierungsaufsatzes MA exakt justieren zu können (in Fig. 7 dargestellt).

Da sich eine Hauptanwendungsform der vorliegenden Erfindung auf die Untersuchung von Hautkrankheiten bezieht und insbesondere die Diagnose und die Operation von Hautkrebs betrifft, kann im Folgenden der Begriff "Objekt" mit der Haut des Patienten gleichgesetzt werden. Andere Ausführungsformen beziehen sich auf andere Untersuchungsobjekte, wie beispielsweise natürliche Proben oder technische Oberflächen.

Unter Bezugnahme auf **Fig. 7** werden die Bauteile der Platine 3 näher erläutert. Neben den oben erwähnten mechanischen Bauteilen umfasst der Markierungsaufsatz MA einen Lichtsignalgeber 2 und eine Platine 3. Der Lichtsignalgeber ist vorzugsweise als Laserpointer ausgebildet. Der Laserpointer 2 befindet sich in einem Stutzen 8, der in einem vordefinierten Winkel an dem Markierungsaufsatz MA ausgebildet ist. Der Lichtsignalgeber 2 dient zur Markierung von Zielpunkten Z auf der Oberfläche des zu untersuchenden Objektes.

Fig. 7a ist eine Draufsicht auf den Markierungsaufsatz MA. Die Platine 3 umfasst einen elektronischen Schaltkreis und einen Lagesensor 4, der zur Lageerfassung des Markierungsaufsatzes MA in allen drei Raumachsen bestimmt ist. Aus der erfassten Lage des Markierungsaufsatzes kann durch eine Berechnungseinheit (nicht dargestellt) eine Geschwindigkeit des Markierungsaufsatzes und damit des Objektivhalters in allen drei Raumachsen bestimmt werden. Der Lagesensor 4 ist vorzugsweise als drei-achsiger Lagesensor ausgebildet und berücksichtigt somit alle sechs Freiheitsgrade im Raum. Der Lagesensor kann entweder eine Beschleunigungsmesseinrichtung sein, die das Gravitationsfeld der Erde auswertet. Alternativ kann der Lagesensor auch einen Gyrator umfassen, der die räumliche Position aus der Auswertung der Coriolis-Kraft bezieht. Alternativ können auch beide vorstehend genannten Alternativen für den Lagesensor kombiniert sein.

Vorzugsweise ist der Lagesensor 4 als integrierter Schaltkreis in die Platine 3 integriert.

Fig. 7b zeigt eine Seitenansicht des Markierungsaufsatzes MA entlang der Schnittlinie A-A in Fig. 7a. Hier ist ersichtlich, dass der Stutzen 8 in einem Winkel in einem vordefinierten Winkel an einer Grundfläche des Markierungsaufsatzes befestigt ist. Vorzugsweise ist der Stutzen 8 einstückig mit dem Markierungsaufsatz ausgebildet. Der Winkel zwischen der Grundfläche des Markierungsaufsatzes A und der Längsachse des Stutzens 8 beträgt vorzugsweise 65°.

Der elektronische Schaltkreis der Platine 3 umfasst des Weiteren eine Aktivierungseinheit 5. Diese Aktivierungseinheit 5 dient dazu, ein Aktivierungssignal an den Laserpointer 2 zur Aktivierung desselben zu senden, falls der Lage- oder Beschleunigungssensor 4 ein Geschwindigkeitssignal erfasst hat, das einen vorkonfigurierbaren Schwellenwert übersteigt. Die elektronische Schaltung umfasst darüber hinaus noch einen oder mehrere Speicherbausteine, z.B. ein RAM und ein Rechenwerk CPU und optional noch weitere Bausteine, wie Lichtsignalsteuerelemente etc.

In einer bevorzugten Ausführungsform der Erfindung wird zwischen zwei Klassen von Geschwindigkeiten des Objektivhalters H differenziert:

    1. Einer schnellen Kippbewegung. Der Schwellenwert liegt hier bei 120°/s. Der Schwellenwert ist vorzugsweise im Vorfeld parametisierbar. Er kann auch während des Betriebs modifiziert werden.

    2. Eine normale oder langsame Bewegung. Diese wird üblicherweise beim Abfahren der Hautoberfläche mit dem konfokalen Mikroskop 10 angewendet.

Durch die Verwendung des Lage- oder Geschwindigkeitssensors 4 wird es möglich, zwischen den beiden Geschwindigkeitsklassen (schnell und normal) zu unterscheiden. Erfindungsgemäß dient das Erfassen eines schnellen Geschwindigkeitssignals (der ersten Klasse), das beispielsweise durch eine schnelle Kippbewegung oder Schwenkbewegung des Objektivhalters H ausgelöst werden kann, zum initiieren eines Wechsels des Betriebsmodus, insbesondere eines Wechsels von einem Aufnahmemodus (der dem normalen Betriebsmodus des konfokalen Mikroskops 10 entspricht) zu einem Markierungsmodus.

Sobald der Objektivhalter H schnell an einer Zielposition Z gekippt wird, ohne die x- und y-Position auf der Haut zu verändern, wird der Markierungsmodus initiiert.

Die verrundete Ausgestaltung der Objektivkappe ermöglicht eine Abrollbewegung des Mikroskops auf der Objektoberfläche ohne die xy-Position vor Bewegungseintritt zu verändern. Beim Zurückkippen des Mikroskops wird wieder die ursprüngliche xy-Position erreicht.

Gemäß der Erfindung ist es grundsätzlich vorgesehen, dass das konfokale Mikroskop 10 in zwei Betriebsmodi betrieben werden kann:

    1. Aufnahmemodus: Der Aufnahmemodus, der dem normalen Betrieb des Mikroskops entspricht und in dem Beleuchtungsinformationen aus einer Fokalebene an einer Zielposition auf der untersuchten Objektoberfläche abgetastet werden. Die abgetasteten Beleuchtungsinformationen werden dann zu einer Bilddarstellung rekonstruiert und auf dem Monitor M dargestellt. In dem Aufnahmemodus befindet sich das Objektiv des Objektivhalters H unmittelbar und direkt auf der Oberfläche der Haut. Dabei ist der Objektivhalter H vorzugsweise so auf der Haut angeordnet, dass eine Längsachse des Objektivhalters H senkrecht auf der Hautoberfläche steht.

    2. Markierungsmodus: Der Markierungsmodus dient zur Markierung von Zielpositionen Z auf der untersuchten Objektoberfläche, insbesondere der Haut. Dabei soll die Zielposition Z mit dem Laserpointer 2 belichtet werden, um so die jeweilige Zielposition Z auf der Haut zu kennzeichnen. Damit kann der Arzt unmittelbar die Zielposition Z auf der untersuchten Haut erkennen. Darüber hinaus erhält er die Möglichkeit, manuell eine Markierung auf der Haut an der belichteten Stelle zu setzen. Desweiteren kann die Objektoberfläche mit einer photoempfindlichen Lösung behandelt sein, die auf die spezifische Wellenlänge des Laserpointers reagiert und einen Farbpunkt auf der Objektoberfläche hinterlässt.

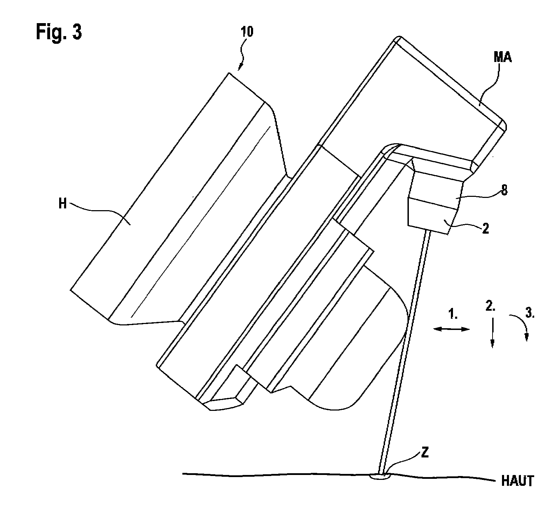

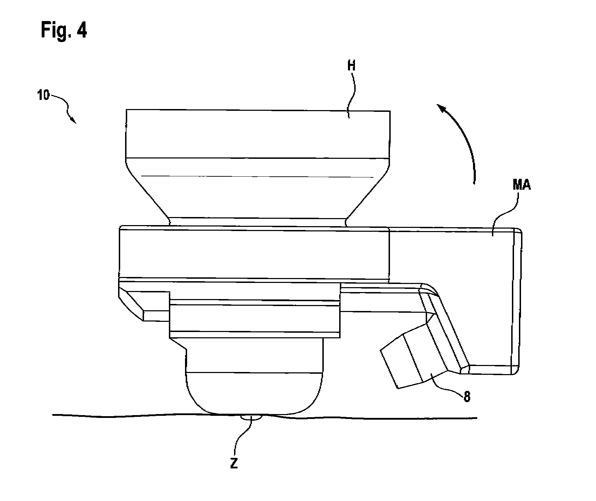

Die beiden Betriebsmodi, Aufnahmemodus und Markierungsmodus, werden im Folgenden detaillierter unter Bezugnahme auf die Figuren 3 und 4 beschrieben.

**Figur 3** zeigt den Betrieb des Mikroskops 10 in dem Markierungsmodus. Wie aus Fig. 3 ersichtlich, ist der Laserpointer 2 aktiviert und sendet einen Markierungsstrahl auf die Hautoberfläche. Der Objektivhalter H befindet sich in einer abgeklappten Stellung, die im Folgenden auch als Markierungsposition bezeichnet wird. Bei der Markierungsposition kann ein Objektiv des Objektivhalters H sich auch beabstandet von der Hautoberfläche befinden und muss nicht zwangsweise auf der Haut aufgesetzt sein. Vorzugsweise ist das System so konfiguriert, dass der Laserpointer 2 erst dann aktiviert wird, wenn sich der Objektivhalter H in der Markierungsposition befindet. Die Markierungsposition ist dabei definiert, dass der von dem Lichtsignalsignalgeber ausgehende Laserstrahl senkrecht auf die Hautoberfläche trifft (in Fig. 2 ist die Markierungsposition somit noch nicht erreicht).

Der Objektivhalter H kann nun jederzeit aus der Markierungsposition und damit aus dem Markierungsmodus heraus bewegt bzw. geführt werden, indem der Objektivhalter H bewegt wird. Die Art bzw. die Geschwindigkeit der Bewegung ist dabei irrelevant. Dies soll in Fig. 3 durch die mit den Pfeilen 1, 2 und 3 gekennzeichnet sein, die mögliche Bewegungsrichtungen des Objektivhalters H repräsentieren (1: Bewegen des Pointers auf der Hautoberfläche zur Suche; 2: Absenken oder Anheben des Objektivhalters; 3: Verkippen des Objektivhalters, um in den Aufnahmemodus zu wechseln).

In einer vorteilhaften Ausführungsform der Erfindung wird davon ausgegangen, dass die Markierungsfunktion dann komplettiert ist, falls der Lichtsignalgeber 2 in dem Markierungsmodus an einer bestimmten Zielposition Z aktiviert worden ist. Wird der Objektivhalter anschließend bewegt, so kann davon ausgegangen werden, dass die Markierungsfunktion abgeschlossen ist und, dass nun in den normalen Aufnahmebetriebsmodus des Mikroskops zurückgewechselt werden soll. In diesem Fall ist es vorgesehen, dass bei Erfassen einer beliebigen Bewegung (diese kann langsam oder schnell sein und somit beiden Geschwindigkeitsklassen angehören) zum Aufnahmemodus gewechselt wird. Alternativ können hier jedoch auch andere Einstellungen gewählt werden, die den Wechsel von dem Markierungsmodus in den Aufnahmemodus einleiten.

Der normale Betriebsmodus, im Folgenden auch als Aufnahmemodus (und gleichbedeutend als Konfokalmodus) bezeichnet, ist in **Fig. 4** dargestellt. Wie aus Fig. 4 ersichtlich, verläuft im Konfokalmodus die Mittellängsachse durch den Objektivhalter H orthogonal zu einer Hautoberfläche, während die Mittellängsachse des Stutzens 8 für den Lichtsignalgeber 2 aufgrund der vordefinierten Geometrie des Markierungsaufsatzes in einem anderen Winkel verläuft. Während des Betriebs des Mikroskops 10 im Konfokalmodus erfolgt eine Erfassung von Beleuchtungsinformationen und eine damit verbundene Darstellung des rekonstruierten Bildes auf dem Monitor M. Um sequentiell unterschiedliche Zielpositionen Z auf der Hautoberfläche zu scannen, wird der Objektivhalter H langsam über unterschiedliche Zielpositionen Z auf der Hautoberfläche geführt. Bei einer langsamen Bewegung des Objektivhalters H ist es eingestellt, dass das Mikroskop 10 weiterhin im Konfokalmodus betrieben wird und damit aktiviert bleibt. Sobald jedoch der Lagesensor 4 erfasst, dass der Objektivhalter H schnell bewegt wird, ist die Bilderfassung des Mikroskops 10 durch das Verlassen der Fokalebene deaktiviert.

Vorzugsweise erfolgt der Wechsel von dem Aufnahme- (bzw. Konfokal-)Modus in den Markierungsmodus durch ein rückwärtiges Abklappen des Objektivhalters an der Zielposition Z auf der Hautoberfläche. "Rückwärtig" meint in diesem Zusammenhang eine Kippbewegung des Objektivhalters auf der Hautoberfläche in eine Richtung, die entgegengesetzt ist zur Mittellängsachse des Markierungsaufsatzes und der Stutzenanordnung 8. Wie in Fig. 4 durch den Pfeil repräsentiert, erfolgt die Schwenkbewegung in diesem Fall nach links oben. Wäre der Stutzen 8 in Fig. 4 auf der linken Seite dargestellt, so wäre die Schwenkbewegung zur Einleitung des Markierungsmodus nach rechts oben. Die schnelle Schwenkbewegung wird somit immer derart ausgeführt, dass die schräg verlaufende Mittellängsachse des Stutzens 8 in einem 90°-Winkel auf die Hautoberfläche an der Position Z trifft.

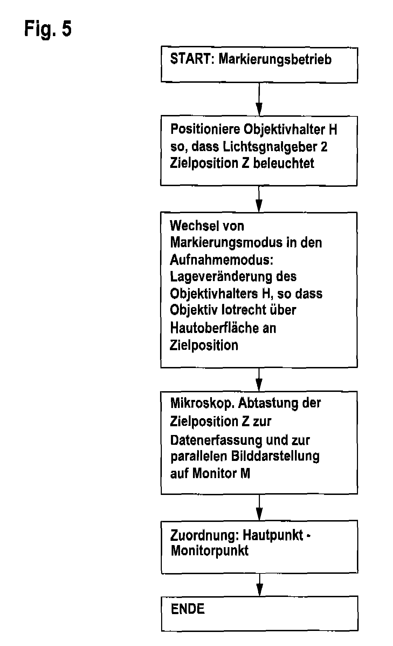

Im Folgenden wird unter Bezugnahme auf **Fig. 5** der Ablauf eines Markierungsbetriebs näher erläutert.

Nach dem Start des Verfahrens wird in einem ersten Schritt der Markierungsmodus ausgewählt.

In einem zweiten Schritt wird der Objektivhalter H so positioniert, dass der Laserpointer 2 die Zielposition Z auf der Hautoberfläche beleuchtet. Anschließend ist es möglich, dass der Arzt den so gekennzeichneten Punkt durch ein Hilfsmittel (z.B. Stift) manuell markiert, um eine bleibende Kennzeichnung zu generieren.

In einem dritten Schritt wird von dem Markierungsmodus auf den Aufnahmemodus gewechselt. Dies erfolgt durch eine Positionsveränderung bzw. Lageveränderung des Objektivhalters H. Vorzugsweise ist der Wechsel von dem Markierungsmodus auf den Aufnahmemodus unabhängig von der Geschwindigkeit der Objektivhalterbewegung. Sobald eine Lageveränderung festgestellt wird, wird der Betriebswechsel initiiert. Der Wechsel hat eine Übergangsphase, die üblicherweise dann endet, wenn sich das Objektiv unmittelbar über der Zielposition Z befindet. Vorzugsweise wird der Objektivhalter dabei so geschwenkt, dass die Längsachse des Objektivhalters senkrecht auf der Objektoberfläche an der jeweiligen Zielposition Z ist.

Im nächsten Schritt kann die mikroskopische Belichtung des Zielpunktes zur Datenerfassung und zur Bilddarstellung auf dem Monitor M durch Widereintritt in die Fokalebene ausgeführt werden. Damit wird eine eineindeutige Zuordnung zwischen einer Referenzposition, die in dem rekonstruierten Bild auf dem Monitor M angezeigt wird (z.B. durch ein Fadenkreuz oder ein eingeblendetes Symbol) und der Zielposition Z auf der Haut, die nach dem Markierungsmodus ebenfalls entsprechend markiert worden ist. Der Arzt kann somit die auf dem Monitor angezeigte Referenzposition eindeutig einer tatsächlichen sichtbaren Zielposition Z auf der Haut zuordnen.

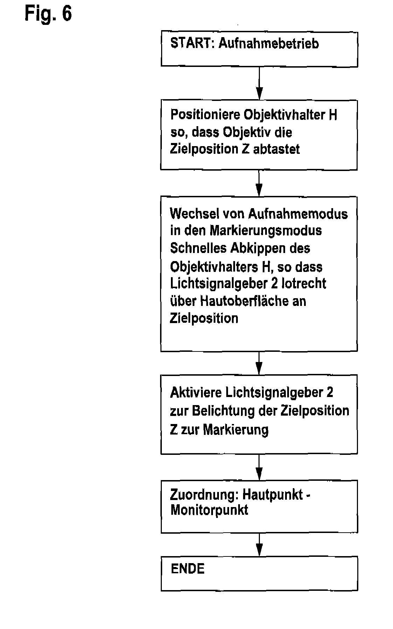

Wie in Fig. 5 dargestellt, kann das Verfahren daraufhin enden. Alternativ ist es auch möglich, dass daraufhin ein normaler Konfokalmodus initiiert wird, um weitere mikroskopische Aufnahmen zu generieren. Der Ablauf für einen Aufnahmemodus ist im Folgenden unter Bezugnahme auf Fig. 6 näher erläutert.

Nach dem Start bzw. Inbetriebnahme des Systems wird der Aufnahmemodus ausgewählt.

In einem zweiten Schritt wird der Objektivhalter H so positioniert, dass das Objektiv eine bestimmte Zielposition Z auf der Hautoberfläche abtastet.

Im nächsten Schritt wird von dem Aufnahmemodus in den Markierungsmodus gewechselt. Dies erfolgt durch schnelles Abklappen des Objektivhalters um ca. 65° entgegen der Richtung, in der der Stutzen 8 angeordnet ist, so dass sich das Objektiv des Lichtsignalgebers senkrecht über der Hautoberfläche an der Zielposition Z befindet. Der Anwender muss dazu normalerweise den Objektivhalter H von sich wegkippen und für den anderen Wechsel in den Aufnahmemodus zu sich her kippen.

Im nächsten Schritt wird der Laserpointer 2 dann aktiviert, wenn die Achse des Laserpointers 2 senkrecht zur Hautoberfläche verläuft.

Anschließend oder parallel zur Markierung durch den Laserpointer 2 kann der Arzt manuell eine zusätzliche Markierung auf der belichteten Zielposition Z ausführen. Auch im Anschluss an den Aufnahmemodus kann eine eineindeutige Zuordnung zwischen der auf dem Bildschirm angezeigten Referenzposition und einer auf der Haut markierten Zielposition Z erreicht werden. Daraufhin kann das Verfahren enden oder nochmals neu initiiert werden. Ebenso ist es möglich, das Verfahren im Markierungsbetrieb fortzuführen.

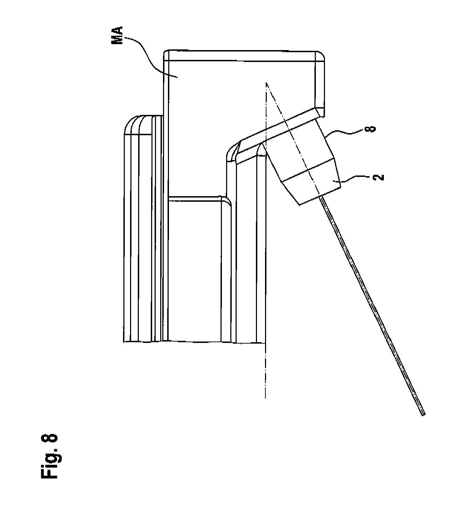

In Fig. 8 zeigt einen Ausschnitt des Markierungsaufsatzes MA, der sich auf eine bevorzugten Ausführungsform der Erfindung bezieht. Darin beträgt der zwischen der Ebene des Markierungsaufsatzes MA und dem Stutzen, der mit dem Bezugszeichen 8 gekennzeichnet ist, 65°. Andere geometrische Verhältnisse erfordern hier einen anderen Winkel.

Gemäß einer weiteren bevorzugten Ausführungsform umfasst das Gesamtsystem, bestehend aus Markierungsaufsatz MA und Mikroskop 10 zusätzlich eine Lasersicherheitsschaltung. Die Lasersicherheitsschaltung kann in dem integrierten Schaltkreis auf der Platine 3 ausgebildet sein oder als Add-on-Modul (z.B. als Firmware-Patch) auf das System des Mikroskops eingespielt werden. Die Lasersicherheitsabschaltung dient zur Abschaltung des konfokalen Lasermessstrahls (des Mikroskops 10) und/oder des Markierungsstrahls des Laserpointers 2, falls der Objektivhalter über einen vorkonfigurierbaren Winkel von der Hautoberfläche weg bewegt wird. Dies soll verhindern, dass der jeweilige Laser aktiviert bleibt, wenn der jeweilige Laserstrahl nicht mehr die Hautoberfläche erreichen kann und beispielsweise den Anwender oder andere Anwender beeinträchtigen würde.

In einer vorteilhaften Weiterbildung der Erfindung ist es vorgesehen, dass das Mikroskop 10 zusätzlich mit zumindest einem weiteren Signalgeber ausgebildet sein kann. Der Signalgeber ist vorzugsweise optisch (beispielsweise eine aktivierte oder deaktivierte LED) und kann jedoch alternativ auch akustisch ausgebildet sein. Die LED-Signalgebung dient dazu, dem Anwender mitzuteilen, ob zumindest einer der Laser aktiv ist. Vorzugsweise ist eine Anzeige vorgesehen, die den konfokalen Laserstrahl betrifft und alternativ oder kumulativ kann eine zweite LED-Anzeige platziert werden, die dem Anwender kennzeichnet, falls der Markierungslaser aktiv ist.

Wie vorstehend beschrieben, basiert die bevorzugte Ausführungsform auf einem Markierungsmodus und einem Aufnahmemodus. Der Markierungsmodus ist eindeutig mit einer Markierungsposition verbunden und der Aufnahmemodus ist eindeutig mit einer Konfokalposition des Objektivhalters H verbunden. In der Markierungsposition verläuft die Längsachse des Stutzens senkrecht auf die Hautoberfläche und in der Konfokalposition verläuft die Längsachse des Objektivhalters senkrecht zur Hautoberfläche. Grundsätzlich kann der Objektivhalter sowohl im Markierungsmodus als auch im Aufnahmemodus bewegt werden (in X- und Y-Richtung) auf der Hautoberfläche, ohne dass der jeweilige Modus notwendigerweise verlassen werden muss. Eine annähernde Markierungsposition ist in Fig. 3 und eine Konfokalposition ist in Fig. 4 dargestellt. In einer Weiterbildung ist es ebenfalls möglich, das Erreichen der jeweiligen Positionen (Markierungsposition und/oder Konfokalposition) durch einen zusätzlichen Signalgeber (z.B. optisches LED) anzuzeigen. In einer alternativen Weiterbildung ist es möglich, Gerätefunktionalitäten über weitere Positionen zu initiieren. Beispielsweise kann es konfiguriert sein, dass das Gerät eine bestimmte Funktionalität (z.B. Power-Off, Selbsttest, Abfrage des Batteriestatus etc.) ausführt, falls sich der Objektivhalter H nicht in der Markierungsposition und nicht in der Konfokalposition befindet. Vorzugsweise kann das Verschwenken bzw. Abklappen des Objektivhalters H in Richtung des Stutzens 8 diese weitere Funktion auslösen. Darüber hinaus ist es möglich, das Auslösen von weiteren Gerätefunktionen durch die Geschwindigkeit der Bewegung des Objektivhalters H auszulösen. Darüber hinaus können beide der vorstehend erwähnten Alternativen auch kombiniert werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Für einen Fachmann ist es insbesondere offensichtlich, dass die Erfindung teilweise oder vollständig in Soft- und/oder Hardware und/oder auf mehrere physikalische Produkte - dabei insbesondere auch Computerprogrammprodukte - verteilt realisiert werden kann.

Bezugszeichenliste

MA    Markierungsaufsatz
10    Mikroskop

1    Halterung
2    Lichtsignalgeber
3    Platine
30    Kontrolleinheit/Controller
4    Beschleunigungssensor
5    Aktivierungseinheit
6    Aufnahmeöffnung
7    Verriegelungsmechanismus
8    Stutzen
9    Justierelement

H    Objektivhalter
Z    Zielposition
R    Referenzposition

**Patentansprüche**

1. Markierungsaufsatz (MA) für ein konfokales Mikroskop (10), der zum Eingriff mit einem Objektivhalter (H) des konfokalen Mikroskops bestimmt ist, mit:

    - Einer mechanischen Halterung (1), die zum Eingriff mit dem Objektivhalter (H) derart bestimmt ist, dass der Markierungsaufsatz (MA) in einer vorbestimmten, ortsfesten Position mit dem Objektivhalter (H) verrastet
    - Zumindest einem Lichtsignalgeber (2), der jeweils in einem vordefinierten Winkel an dem Markierungssaufsatz (MA) angeordnet ist,
    - Einer Platine (3) mit einem elektronischen Schaltkreis mit:

        o Einem drei-achsigen Lagesensor (4), der zur Lageerfassung des Markierungsaufsatzes (MA) in allen drei Raumachsen und daraus ableitbaren Geschwindigkeiten des Objektivhalters (H) in allen drei Raumachsen bestimmt ist
        o Aktivierungseinheit (5), die dazu bestimmt ist, ein Aktivierungssignal an den Lichtsignalgeber (2) zur Aktivierung des Lichtsignalgebers (2) zu senden, falls das erfasste Geschwindigkeitssignal einen vorkonfigurierbaren Schwellenwert übersteigt.

2. Markierungsaufsatz (MA) nach Patentanspruch 1, bei dem der Lichtsignalgeber (2) ein Laserpointer ist.

3. Markierungsaufsatz (MA) nach einem der vorstehenden Patentansprüche, bei dem das konfokale Mikroskop (10) als bewegliches Handgerät ausgebildet ist.

4. Markierungsaufsatz (MA) nach einem der vorstehenden Patentansprüche, bei dem die mechanischen Halterung (1) eine Aufnahmeöffnung (6) umfasst mit einem lichten Durchmesser, der in etwa dem Außendurchmesser des Objektivhalters (H) entspricht, und einen Verriegelungsmechanismus (7) zur Fixierung des Markierungsaufsatzes (MA) auf dem Objektivhalter (H) in Bezug auf eine Längsachse des Objektivhalters (H).

5. Markierungsaufsatz (MA) nach einem der vorstehenden Patentansprüche, bei dem der Lichtsignalgeber (2) in einem Stutzen (8) angeordnet ist, wobei der Stutzen (8) in dem vordefinierten Winkel einstückig mit dem Markierungsaufsatz (MA) ausgebildet ist.

6. Konfokales Mikroskop (10) mit einem Markierungsaufsatz (MA) nach einem der vorstehenden Patentansprüche zur Markierung von zumindest einer Zielposition (Z) und zur Zuordnung einer auf einer Objektoberfläche durch den Lichtsignalgeber (2) belichteten Zielposition (Z) und einer Referenzposition (R) in einer auf einem Monitor (M) dargestellten Bilddarstellung.

7. Verfahren zum Betreiben eines konfokalen Mikroskops (10) mit einem Objektivhalter (H) in:

    - einem Aufnahmemodus, in dem Beleuchtungsinformationen aus der Objektoberfläche an zumindest einer Zielposition (Z) abgetastet werden und zu einer auf einem Monitor (M) darstellbaren Bilddarstellung rekonstruiert werden und
    - einem Markierungsmodus, in dem eine Bilderfassung des konfokalen Mikroskops (10) deaktiviert ist und in dem ein Lichtsignalgeber (2) aktiviert ist, der die abgetastete Zielposition (Z) auf der untersuchten Objektoberfläche belichtet, wobei ein Wechsel zwischen Aufnahmemodus und Markierungsmodus des konfokalen Mikroskops (10) durch eine schnelle Verschwenkbewegung des Objektivhalters (H) erfolgt.

8.  Verfahren nach Patentanspruch 7, wobei für unterschiedliche Zielpositionen (Z) ein Wechsel zwischen den beiden Betriebsmodi ohne ein Umrüsten des konfokalen Mikroskops (10) ausgeführt wird.

9.  Verfahren nach Patentanspruch 7 oder 8, wobei das Verfahren zum Positionieren des Objektivhalters (H) an der zuvor auf der Objektoberfläche durch Belichtung markierten Zielposition (Z) dient, mit folgenden Verfahrensschritten:

    - Betreiben des konfokalen Mikroskops (10) in dem Markierungsmodus zur Belichtung der Zielposition (Z)
    - Wechseln des Betriebsmodus in den Aufnahmemodus durch Lageveränderung des Objektivhalters (H) in eine Aufnahmeposition und automatisches Aktivieren des konfokalen Mikroskops (10).

10. Verfahren nach Patentanspruch 7 oder 8, wobei das Verfahren zum Markieren einer zuvor abgetasteten Zielposition (Z) dient, mit folgenden Verfahrensschritten:

    - Betreiben des konfokalen Mikroskops (10) in dem Aufnahmemodus zur Abtastung an der Zielposition (Z)
    - Wechseln des Betriebsmodus in den Markierungsmodus durch Verschwenken des Objektivhalters (H) in eine Markierungsposition und
    - Automatisches Aktivieren des Lichtsignalgebers (2) zum Applizieren von Lichtsignalen zum Markieren der zuvor abgetasteten Zielposition (Z), falls der Lichtsignalgeber (2) in etwa orthogonal zur Objektoberfläche positioniert ist.

11. Verfahren nach einem der vorstehenden Verfahrensansprüche, bei dem der Wechsel zwischen den beiden Betriebsmodi durch das Erfassen von Positionssignalen des Objektivhalters (H) mittels eines drei-achsigen Lagesensors (4) erfolgt, wobei aus den erfassten Positionssignalen ein Geschwindigkeitssignal des Objektivhalters (H) berechnet wird, so dass bei Überschreiten des berechneten Geschwindigkeitssignals über einen parametrierbaren Schwellwert automatisch in den Markierungsmodus gewechselt wird.

Fig. 1

Fig. 2

Fig. 3

Fig. 4

## Fig. 5

START: Markierungsbetrieb

↓

Positioniere Objektivhalter H so, dass Lichtsgnalgeber 2 Zielposition Z beleuchtet

↓

Wechsel von Markierungsmodus in den Aufnahmemodus: Lageveränderung des Objektivhalters H, so dass Objektiv lotrecht über Hautoberfläche an Zielposition

↓

Mikroskop. Abtastung der Zielposition Z zur Datenerfassung und zur parallelen Bilddarstellung auf Monitor M

↓

Zuordnung: Hautpunkt - Monitorpunkt

↓

ENDE

**Fig. 6**

START: Aufnahmebetrieb

Positioniere Objektivhalter H
so, dass Objektiv die
Zielposition Z abtastet

Wechsel von Aufnahmemodus
in den Markierungsmodus
Schnelles Abkippen des
Objektivhalters H, so dass
Lichtsignalgeber 2 lotrecht
über Hautoberfläche an
Zielposition

Aktiviere Lichtsignalgeber 2
zur Belichtung der Zielposition
Z zur Markierung

Zuordnung: Hautpunkt -
Monitorpunkt

ENDE

**Fig. 7a**

**Fig. 7b**

65°

A - A

EP 2 711 758 A1

Fig. 8

Europäisches
Patentamt

European
Patent Office

Office européen
des brevets

**EUROPÄISCHER RECHERCHENBERICHT**

Nummer der Anmeldung

EP 12 18 5535

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2005/109068 A1 (LEICA MICROSYSTEMS SCHWEIZ AG [CH]; SANDER ULRICH [CH]) 17. November 2005 (2005-11-17) * Seite 8, Zeile 1 - Seite 9, Zeile 5; Abbildung 1 * * Seite 13, Zeilen 24-31 * ----- | 1-11 | INV. G02B21/00 A61B5/00 G02B21/34 |
| A,D | WO 98/17166 A2 (LUCID TECH INC [US]) 30. April 1998 (1998-04-30) * Seite 6, Zeile 8 - Seite 7, Zeile 18; Abbildung 1 * ----- | 1-11 | |
| A,D | WO 00/15105 A1 (LUCID INC [US]; ZAVISLAN JAMES M [US]; GREENWALD ROGER J [US]) 23. März 2000 (2000-03-23) * Seite 15, Zeile 26 - Seite 16, Zeile 21; Abbildung 9 * ----- | 1-11 | |
| A | WO 00/69335 A1 (ELBIT SYSTEMS LTD [IL]) 23. November 2000 (2000-11-23) * Seite 32, Zeile 21 - Seite 33, Zeile 2 * ----- | 1-11 | RECHERCHIERTE SACHGEBIETE (IPC) |
| A | WO 2006/124800 A2 (LUCID INC [US]; FOX WILLIAM J [US]; GRODEVANT SCOTT R [US]; DISTASIO C) 23. November 2006 (2006-11-23) * das ganze Dokument * ----- | 1-11 | G02B A61B |
| A | WO 2004/104645 A2 (LUCID INC [US]; FOX WILLIAM J [US]; DISTASIO CHRISTOPHER C [US]; GRODE) 2. Dezember 2004 (2004-12-02) * das ganze Dokument * ----- | 1-11 | |

Der vorliegende Recherchenbericht wurde für alle Patentansprüche erstellt

| Recherchenort | Abschlußdatum der Recherche | Prüfer |
|---|---|---|
| München | 14. März 2013 | Hambach, Dirk |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 12 18 5535

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-03-2013

| Im Recherchenbericht angeführtes Patentdokument | Datum der Veröffentlichung | Mitglied(er) der Patentfamilie | Datum der Veröffentlichung |
|---|---|---|---|
| WO 2005109068 A1 | 17-11-2005 | DE 102004022330 B3 | 20-10-2005 |
| | | JP 4642842 B2 | 02-03-2011 |
| | | JP 2007536584 A | 13-12-2007 |
| | | US 2007216998 A1 | 20-09-2007 |
| | | WO 2005109068 A1 | 17-11-2005 |
| WO 9817166 A2 | 30-04-1998 | AU 4669697 A | 15-05-1998 |
| | | EP 1011441 A2 | 28-06-2000 |
| | | US 6424852 B1 | 23-07-2002 |
| | | US 7225010 B1 | 29-05-2007 |
| | | US 2002151782 A1 | 17-10-2002 |
| | | US 2005171441 A1 | 04-08-2005 |
| | | US 2007287921 A1 | 13-12-2007 |
| | | WO 9817166 A2 | 30-04-1998 |
| WO 0015105 A1 | 23-03-2000 | AU 5923299 A | 03-04-2000 |
| | | EP 1113749 A1 | 11-07-2001 |
| | | WO 0015105 A1 | 23-03-2000 |
| WO 0069335 A1 | 23-11-2000 | AU 4607600 A | 05-12-2000 |
| | | CA 2373295 A1 | 23-11-2000 |
| | | EP 1185199 A1 | 13-03-2002 |
| | | EP 2204123 A1 | 07-07-2010 |
| | | JP 5005128 B2 | 22-08-2012 |
| | | JP 2003520062 A | 02-07-2003 |
| | | US 6233476 B1 | 15-05-2001 |
| | | WO 0069335 A1 | 23-11-2000 |
| WO 2006124800 A2 | 23-11-2006 | EP 1895905 A2 | 12-03-2008 |
| | | US 2009097108 A1 | 16-04-2009 |
| | | WO 2006124800 A2 | 23-11-2006 |
| WO 2004104645 A2 | 02-12-2004 | CN 1791822 A | 21-06-2006 |
| | | CN 102135661 A | 27-07-2011 |
| | | EP 1636625 A2 | 22-03-2006 |
| | | EP 2278375 A1 | 26-01-2011 |
| | | US 2006274407 A1 | 07-12-2006 |
| | | US 2008239474 A1 | 02-10-2008 |
| | | WO 2004104645 A2 | 02-12-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006018777 W
- WO 0015105 A